# EUROPEAN PATENT SPECIFICATION

(11) **EP 1 487 285 B1**
(45) Date of publication and mention of the grant of the patent: **19.12.2007**
(21) Application number: 03744909.7
(22) Date of filing: 24.03.2003
(51) Int. Cl.: A23L 1/29, A23L 1/30, A23L 1/305, A23L 1/302, A23L 1/304, A23L 1/09

(54) **ALERTNESS BAR**
WACHSAMKEITSRIEGEL
BARRE FAVORISANT LA VIVACITE INTELLECTUELLE

(30) Priority: 22.03.2002 GB 0206793
(43) Date of publication of application: 22.12.2004
(73) Proprietor: Galactogen Products Limited, Toronto, Ontario M6J 3L9 (CA)
(72) Inventor: KING, Roderick, Frederick, Gerardus, Joseph, Otley LS21 2RG (GB); LESTER, Simon, Toronto, Ontario M4N 1P3 (CA)
(74) Representative: Moore, Christopher Mark
(86) International application number: PCT/GB2003/001241
(87) International publication number: WO 2003/079818

(56) References cited:
- WO-A-01/28360
- WO-A-01/78522
- WO-A-98/17286
- WO-A-99/65335
- GB-A- 823 556
- US-A1- 2002 150 607

## Description

This invention relates to a food product particularly but not exclusively to an edible bar, gum or chew to improve the creation and maintenance of an alert state or to reverse a previously lowered state of a consumer. The product may serve to improve the mental and physical alertness of a user leading to a maintenance and/or improvement of cognitive function. The product may also provide an improvement of alertness, concentration, reaction time, response time and energy levels. An improvement of memory, learning, word recall and mood may also be achieved. Preferred products provide enhanced maintenance and repair of cell membranes.

The product may comprise an edible bar, gum, chew or powder composition for making into a drink.

There is a need for a formulation containing efficacious substances that correctly address energy and substrate needs for the enhancement or maintenance of alertness in situations where either acute or progressive reductions in alert state are evident or expected. This may occur at any time during the daily lifestyle of an individual and will depend on previous current and future actitives.

Previously known products are only able to provide limited solutions to the problems of achieving a balance between mental and physical states. It is well known that fuels, endogenous energy reserves and exogenous substrates can provide energy to support general physical activity and that fatigue can arise from shortfalls of such substances. It is also known that alertness can be improved by consumption of active ingredients to be found in coffee, tea and other herbal beverages to limit or offset fatigue. Alertness can be viewed as that state arising when central and peripheral nervous system pathways are fully active such that there is no central or peripheral fatigue. In such a situation alertness is the combination of complete functional neuronal activity coupled with full functional ability to provide energy. However an empirical combination of ingredients is prone to failure in that combinations do not always act summatively and may even act antagonistically. Further, neglect of the fabric with which alertness is effected i.e. nerves and associated tissue, may lead to situations in which use of effective substances is sub-optimal because cell and neuronal function has been reduced, either by centrally mediated potentials or reduced metabolic maintenance. WO9817286 describes therapeutic food for treatment of diabetic patients to diminish fluctuations in blood sugar levels comprising chocolate (caffeine), fat, proteine, phosphadityl choline and carbohydrates.

Exercise places considerable demands on the use of fuels to provide energy for working muscle and other tissues, and building blocks to maintain cellular function in all tissues to support normal body function. These fuels and building blocks can be sourced from both endogenous and exogenous substrates primarily in the form of glycogen in liver and muscle, glucose, fatty acids and amino acids in plasma, lipids in adipose tissue and muscle, and proteins. Tissues are unable to function without at least one source of fuel and are only able to function maximally when more than one source is available. Even then there are restrictions on both intensity and endurance of what are normal activities because each fuel source is only of finite size. It is not surprising that the status of the human body changes between meals and in periods when reserves become depleted. It is at such a time that attention declines and the state of alertness is reduced. Alertness may decline, particularly during repetitive activities, and can be severely restricted if either or both of the exogenous fuel supplies to provide substrates or endogenous reserves previously built up during recovery are inadequate. The provision of specific nutrients is beneficial because these substrates add to those used from the body's endogenous sources. The provision of selected nutrients during such times can improve alertness. This is related both to the repletion of body reserves and the maintenance of healthy tissue. Limitations in the prior art restrict the design of the most efficacious formulations for these situations because antagonisms between nutrients and new synergies were not known. This invention addresses and seeks to overcome these limitations.

According to the present invention a nutritional composition for enhancing alertness comprises carbohydrate including galactose, fat, protein, caffeine and optional further ingredients;
wherein the ratio of carbohydrate to protein is in the range of 2:1 to 4:1;
and wherein the fat includes an effective quantity of phospholipid selected from phosphatidyl choline, phosphatidyl serine and mixtures thereof.

In a preferred embodiment the ratio of carbohydrate to protein is in the range 2.5:1 to 3.5:1, preferably 2.7:1 to 3.2:1, more preferably 3:1 to 1.

The carbohydrate may be selected from galactose, fructose, sucrose, maltodextrin, refined and raw cane sugar, hydrolysed lactose and mixtures thereof. The composition may also include starches and complex polysaccharides such as are present in nuts, seeds and fruit.

It is advantageous to fortify the protein content of the bar with glutamine dipeptide. 5-15% of the total protein content may comprise glutamine dipeptide. Glutamine is both a substrate for cellular tissue and is useful for maintenance and enhancement of immune function.

Caffeine may be provided as purified caffeine, a natural substance containing caffeine, for example, guarana or mixtures thereof. An amount of 0.010 to 0.200%, preferably 0.025 to 0.100%, more preferably 0.075% may be employed.

A preferred amount of phospholipid is 0.2 to 1.0%, more preferably 0.25 to 0.3%. The use of a mixture of phosphatidyl serine and phosphatidyl choline is preferred, preferably in a ratio in the range 1:3 to 3:1. Generally equal amounts are especially preferred.

Phosphatidyl serine is especially important as a key phospholipid in the neuronal cell membrane and may be categorised as a brain nutrient. Together with galactose, these phospholipids are crucial to functionality of neuronal tissue. Of key importance, neuronal tissue in the brain is characterised by a class of compounds called cerebrosides, the most prevalent of which is a glycosphingolipid in which galactose forms the head group. (galacto-cerebrosides). Galactose is also found in the oligosaccharide parts of structural membrane glycoproteins.

Phosphatidyl serine from bovine and soy sources is superior to that from egg sources. For this reason the formulation of products of this invention may advantageously use at least 50% of phosphatidyl serine and phosphatidyl choline sourced from bovine and/or soy phospholipids. Use of soy products allows production of a vegetarian product.

The fat component, preferably includes about 5 to 10% mono-unsaturated fatty acids (MUFA) and about 10 to 15% poly-unsaturated fatty acids (PUFA).

The use of ingredients to supply beneficial proportions of omega-6 and omega-3 fatty acids is preferred. Fish oils, vegetable oils or a mixture of these may be used. Vegetable oils (wheat germ, flaxseed, safflower, soybean, cotton seed, sesame and sunflower) are good sources of linoleic acid (omega-6). Vegetable oils (flaxseed, soybean, walnut and wheat germ) are good sources of alpha linolenic acid (omega-3). Fish oils are also rich sources of these essential fatty acids. A combination of flaxseed oil (high in PUFA and linolenic acid) and wheat germ oil (high in PUFA, linoleic acid and vitamin E) is made with evening primrose oil (high in gamma linolenic acid) and starflower oil (borage oil, high in gamma linolenic acid). This may be used as a vegetarian alternative to use of fish oil (such as cod liver oil). The ideal ratio of omega-6 to omega-3 content may be 4-10:1. Thus within products of this invention in which the ratio of MUFA to PUFA may be between 1:3 to 1:1, that part that is PUFA may contain a ratio between 4-10:1 of omega-6 to omega-3 fatty acids from any or a combination of all the sources described.

The composition may be formulated as a chewable bar, biscuit, cookie or cake. Sugar confectionery, gum or other tablet or cereal composition may also be provided. Liquid drinks or powder or granular blends for rehydration into drinks may also be employed.

Percentages and other proportions used in the specification are by dry weight unless indicated otherwise. Percentages and proportions may be selected from ranges given to total 100%.

Macronutrients referred to below are carbohydrates, fat and protein, that is excluding minerals, stabilisers, fibre, caffeine and other ingredients.

Use of a mixture of carbohydrates containing galactose or fructose may ensure a low glycaemic index. Galactose and fructose are non-insulogenic. The preferred mixture of simple carbohydrates and sugars with maltodextrins and complex starches may ensure sustained delivery of energy. The sustained delivery of energy may be possible because the balance of non-insulogenic sugars, especially galactose and fructose together with disaccharides and other complex carbohydrates may provide rapid and sustained phases of absorption and assimilation of substrates for maintenance of blood glucose concentration.

The most preferred ratio of carbohydrate to protein is 3:1. This ratio is particularly effective for alertness. Higher proportions of carbohydrate may induce drowsiness.

The provision of both soluble and insoluble fibre in the formulation may act to aid digestion, absorption and assimilation of carbohydrates to ensure phased and sustained delivery of energy. Pectin may be included. An amount of 2-10%, preferably 5-8% of fibre may be used of which up to 50% may be pectin. ,

Antioxidants are an important part of the ingredients in the formulation of "Alertness" bars. Vitamin E protects against lipid peroxidation, Vitamin C quenches free radicals, Green Tea extract contains useful antioxidants and also may supply part of the caffeine (this needs to go in ingredients in table of examples).

The proteins include both whey protein for rapid provision of amino acids and casein for longer term sustained provision of amino acids. Galactose, phosphatidyl choline and phosphatidyl serine provide percursors for the maintenance and synthesis of cell membranes, especially neuronal tissue. Antioxidants protect these membranes and the fat composition in the bar is intended to limit lipid peroxidation. The provision of phospholipids and other ingredients in conjunction specifically with caffeine to act as a stimulant together with the appropriate energy to ensure stability of blood glucose and energy supply act synergistically to effect an increase in alertness, prevention of alertness decline and maintenance of an enhanced state of alertness.

Thus to effect beneficial and significant improvements in alertness it is desirable to use compositions of exogenous ingredients that provide one or more:
1) permit sustained and effective sources of fuels and building blocks for tissue maintenance, especially those tissues associated with alertness;
2) permit the most effective presentation of fuels to the body tissues;
3) effect maintenance or enhancement of alertness acutely following digestion and assimilation;
4) facilitate the maximum alertness potential during normal lifestyle; and
5) provide a synergy exclusive to the use of these ingredients.

The carbohydrate:protein ratio should be within the range 2:1 to 4:1 by energy or mass. This is 48 to 57.6% carbohydrate, 24 to 14.4% protein, 28% fat on an energy basis (ie by proportion of the total macronutrient energy available). Since fat has approximately twice the calorific value of carbohydrate or protein, this would equate to 56.9 to 68.2% carbohydrate 28.4 to 17.1% protein, 14.7% fat on a mass basis of macronutrients (ie by dry weight of macronutrients but not by weight of bar containing variable amount of moisture). However the ratio of carbohydrate to protein was 3:1 with variation of fat from 15% to 45% on an energy basis or 7.3% to 26.7% on a mass basis.

The carbohydrate:protein ratio in the prefered embodiment is 3:1 on an energy basis or by weight. Preferably this is 54% carbohydrate, 18% protein, 28% fat on an energy basis (ie by proportion of the total macronutrient energy available). Since fat has approximately twice the calorific value of carbohydrate or protein, this would equate preferably to 64% carbohydrate, 21.3% protein, 14.7% fat on a mass basis of macronutrients (ie by dry weight of macronutrients but not by weight of bar containing variable amount of moisture).

The specific carbohydrates include all those above (both as pure saccharides or wholesome organic nutrients) to yield or produce by digestion or otherwise monosaccharides to include galactose, glucose and fructose. The ratio of content (mono, di, oligo, poly and complex saccharides of each type, % by weight of carbohydrate content) may be 14% to 59% by weight galactose yielding, 71% to 29% by weight glucose yielding, 15% to 12% fructose yielding (to achieve 100% by weight of carbohydrate content). The prefered embodiment is 30% by weight galactose yielding, 60% by weight glucose yielding, 10% by weight fructose yielding.

The percentage of galactose relative to total carbohydrate may be in the range of 50-98%.

At least 90% of the galactose content should be in the form of monosaccharide or free galactose.

Within the proportions described, that part as galactose is largely in the form of monosaccharide (14% to 59% by weight of total carbohydrate content) and that part yielding glucose (71 %-29% by weight of total carbohydrate content) is a mixture of monosaccharide and higher saccharides of ratio 1:4 (20% by weight of glucose yielding content as free glucose, and 80% by weight of glucose yielding content as higher saccharides) such that in the total carbohydrate mixture the amount of free glucose as monosaccharide is no more than 40% of the galactose content and no more than 20% of the glucose yielding higher saccharide content. A preferred embodiment comprises 29.4% galactose, 11.8% free glucose, 47% higher glucose yielding saccharides and 11.8% fructose.

Within the proportions described, 10% to 50% by mass of the fat content, (given above may range from 7.3% to 26.7% by weight of macronutrients), may be in the form of monounsaturated (MUFA) and polyunsaturated (PUFA) fatty acids, ie the combined proportions of MUFA and PUFA maybe 0.73% to 13.35% of macronutrients wherein MUFA may be 0.18% to 6.68% and PUFA may be 0.36% to 10% of macronutrients such that the sum of MUFA and PUFA may be in the range 0.73% to 13.35% (ie a resulting ratio of MUFA to PUFA between 1:3 to 1:1).

The proportion of protein to carbohydrate (total as above), is preferred at 1:3 by energy. 20 to 70% of the protein content, preferably 50% should be whey and casein of variable proportion, (casseinates may be used in part). 80 to 30% of the protein content may be from natural products such as nuts, seeds and flour (e.g. soy flour), also from soy or rice protein. Part of the protein content may be added as a hydrolysate e.g. wheat protein hydrolysate.

Flavour as necessary e.g. lemon, lime, blackcurrant, orange, citrus, cranberry, chocolate. The mixture is formulated such that these constituents give bars that range from chewy to dry according to taste. Water content may be adjusted accordingly. For example, a water content may range from 2 to 40%, preferably 10 to 30%, more preferably 15 to 20%. Suggested size and mass for energy bars could range from 50g to 150g. A typical bar may be 100g of mixed ingredients.

The invention is further described by means of example but not in any limitative sense.

The following table lists ingredients of preferred products in accordance with this invention.

### EXAMPLES

| **Ingredient** | **Weight/%** | | | | | | | | |
|---|---|---|---|---|---|---|---|---|---|
| | **A** | **B** | **C** | **D** | **E** | **F** | **General Range Lower limit-Upper limit** | **Prefered range** | **Model Bar** |
| Galactose Part of the galactose requirement may be in the form of date or fig pastes or syrups. Part of the requirement may be in the form of fruit concentrates. | 5 | 20 | - | 5 | 5 | - | 5-37.5 | 10-30 | 14.4 |
| Polysaccharides Complex carbohydrates may be sourced from a combination in variable proportion of cornstarch, seeds, flour, rice, rolled oats, fruit and nuts. To include dried and preserved fruit. | 20 | 10 | 20 | 30 | 24 | 24 | 5-30 | 10-25 | 14.4 |
| Maltodextrin | 12.5 | 7.5 | 12.5 | 2.5 | 12.5 | 12.5 | 3-30 | 4.5-15 | 4.8 |
| Raw Cane Sugar | 2 | 2 | 2 | 2 | 2 | 2 | 1-10 | 2-8 | 2.9 |
| Fructose Part of the fructose requirement may be in the form of HFCS or fruit concentrates. | 3 | 3 | 3 | 3 | 3 | 3 | 0.5-10 | 0.75-7 | 0.96 |
| Refined Cane Sugar | 5 | 5 | 5 | 5 | 5 | 5 | 1-20 | 1.75-10 | 1.92 |
| Glucose Part of the requirement could be in the form of brown rice syrup. | 5 | 5 | - | 5 | 5 | - | 1.5-15 | 1.75-10 | 1.92 |
| Hydrolysed Lactose being a Gal/Glu Syrup or derivative thereof with approx 40-50% galactose and variable glucose and fructose. | - | - | 10 | - | - | 10 | 5-50 | 5-40 | 9.6 |
| Lactose Lactose may be part of hydrolysed lactose syrup. | - | - | - | - | I | 1 | 0.5-2 | 0.75-1.5 | 0.96 |
| Fat (proportions of Monounsaturated fatty acids (MUFA) and Polyunsaturated acids (PUFA) are 5-10% and 10-15% respectively of the total energy content) Part of the fat requirement to supply essential fatty acids may be in the form of the oils | 10 | 10 | 10 | 10 | 10 | 10 | 5-30 | 7-15 | 9.6 |
| from flaxseed, sunflower, fish and olive or other vegetable or animal oils. (supply of omega 3/6 fatty acids). | | | | | | | | | |
| Conjugated Linoleic Acid (CLA) | - | - | - | - | 1 | 1 | 0.5-2 | 1-1.5 | 1 |
| Fibre (Sol&Insol) e.g. oat bran Part of the fibre content may be in the form of pectin. | 5 | 5 | 5 | 5 | 5 | 5 | 2-10 | 5-8 | 6.7 |
| Vitamins A vitamin mix may be used to supply the complete range of vitamins. | 10-100% RDA | 10-100% RDA | 10-100% RDA | 10-100% RDA | 10-100% RDA | 10-100% RDA | 10-100%RD A | 40-60% RDA | 50% RDA |
| Protein (50% of which is whey and casein of variable proportion, casseinates may be used in part). The proportion of protein to carbohydrate (total as above, is 1:3 by energy. 50% of protein may be from natural products such as nuts, seeds and flour (e.g. soy flour), also from soy or rice protein. Part of the protein content may be added as a hydrolysate e.g. wheat protein hydrolysate. | 17.5 | 17.5 | 17.5 | 17.5 | 17.5 | 17.5 | 6-30 | 12-24 | 17.3 |
| Flavours | 1 | 1 | 1 | 1 | 1 | 1 | 1 | 1 | 1 |
| Minerals To include Fe, Zn,Ca, and other essential minerals. | 0-100% RDA | 0-100% RDA | 0-100% RDA | 0-100% RDA | 0-100% RDA | 0-100% RDA | 10-100%RD A | 40-60% RDA | 50% RDA |
| Stabilisers To include citrate, phosphate and other buffer systems. | 2 | 2 | 2 | 2 | 2 | 2 | 2 | 2 | 2 |
| Water | 11 | 11 | 11 | 11 | 5 | 5 | 2-20 | 5-15 | 10 |
| | | | | | | | | | |
| | | | | | | | | | |
| Phosphatidyl serine Part of the requirement may be in the form of egg or soy phospholipid | 0.150 | 0.150 | 0.150 | 0.150 | 0.150 | 0.150 | 0.050-0.350 | 0.125-0.175 | 0.15 |
| Phosphatidyl choline Part of the requirement may be in the form of egg or soy lecithin | 0.125 | 0.125 | 0.125 | 0.125 | 0.125 | 0.125 | 0.050-0.250 | 0.100-0.175 | 0.125 |
| Vitamin E | 200 IU | 200 IU | 200 IU | 200 IU | 200 IU | 200 IU | 50-400 IU | 150-250 IU | 200 IU |
| Vitamin C | 0.125 | 0.125 | 0.125 | 0.125 | 0.125 | 0.125 | 0.050- | 0.100- | 0.150 |
| | | | | | | | 0.500 | 0.200 | |
| Caffeine Added as either/or a mixture of caffeine and/or a natural substance containing caffeine e.g. Guarana or green tea | 0.100 | 0.100 | 0.025 | 0.025 | 0.050 | 0.050 | 0.010-0.200 | 0.025-0.100 | 0.075 |
| Other antioxidants. e.g.Green tea ext, soy isoflavones etc. | 0.1 | 0.1 | 0.1 | 0.1 | 0.1 | 0.1 | 0.1-0.2 | 0.13-0.17 | 0.15 |

Ingredients are given by mass (g or mg). Vitamins by mg or IU or %RDA, variation will occur depending on specific type of bar.

Examples above are based on 100g ingredients. The suggested bar is 50-60g.

## Claims

1. A nutritional composition for enhancing alertness comprising:
carbohydrate including galactose,
fat,
protein,
caffeine,
and optional further ingredients,
wherein the ratio of carbohydrate to protein is in the range of 2:1 to 4:1 by weight, and wherein the fat includes an effective quantity of phospholipids selected from: phosphatidyl choline and phosphatidyl serine and mixtures thereof.

2. A composition as claimed in claim 1 wherein the ratio is in the range 2.5=1 to 3.5:1.

3. A composition as claimed in claim 2 wherein the ratio is in the range 2.7:1 to 3.2:1.

4. A composition as claimed in claim 3 wherein the ratio is 3:1.

5. A composition as claimed in any preceding claim wherein the carbohydrate is selected from: galactose, fructose, sucrose, moltodextrin, refined and raw cane sugar, high fructose corn syrup, hydrolysed lactose, starches and complex polysaccharides and mixtures thereof.

6. A composition as claimed in claim 5 wherein at least 90% of the galactose content is as the monosaccharide.

7. A composition as claimed in any preceding claim wherein the caffeine is selected from caffeine, natural products containing caffeine and mixtures thereof.

8. A composition as claimed in any preceding claim wherein the amount of phospholipid is 0.1-2.0%.

9. A composition as claimed in claim 8 wherein the amount of phospholipid is 0.2-1.0%.

10. A composition as claimed in claim 9 wherein the amount of phospholipid is 0.2-0.3%.

11. A composition as claimed in any preceding claim wherein the ratio of phosphatidyl choline to phosphatidyl serine is 1:3-3:1.

12. A composition as claimed in claim 11 wherein the ratio is 1:1.

13. A composition as claimed in any preceding claim wherein the fat component includes 5-10% mono-unsaturated fatty acids and 10-15% polyunsaturated fatty acids.

14. A composition as claimed in claim 13 wherein the proportion of omega 6 fatty acids to omega 3 fatty acids is in the range of 4-10:1.

15. A composition as claimed in any preceding claim wherein the ratio of monosaccharide to higher saccharide is 1:2-1:8.

16. A composition as claimed in claim 15 wherein the ratio of monosaccharide to higher saccharides is 1:3-1:6.

17. A composition as claimed in claim 16 wherein the ratio of monosaccharide to higher saccharides is 1:4.

18. A composition as claimed in any preceding claim wherein the protein is selected from: whey, casein, protein hydrolysate and mixtures thereof.

19. A composition as claimed in any preceding claim wherein the protein includes glutamine dipeptide.

20. A composition as claimed in claim 19 wherein the amount of glutamine dipeptide is in the range of 5-15%.

21. A composition as claimed in any preceding claim including an antioxidant.

22. A composition as claimed in claim 21 wherein the antioxidant is selected from ascorbic acid, green tea extracts and mixtures thereof.

## Patentansprüche

1. Nährzusammensetzung zur Erhöhung der Aufmerksamkeit, umfassend:
Kohlenhydrat, einschließlich Galactose,
Fett,
Protein,
Koffein,
und gegebenenfalls weitere Bestandteile,
wobei das Verhältnis von Kohlenhydrat zu Protein im Verhältnis von 2:1 bis 4:1 nach Gewicht liegt und wobei das Fett eine effektive Menge an Phospholipiden, ausgewählt aus Phosphatidylcholin und Phosphatidylserin und Mischungen davon, einschließt.

2. Zusammensetzung nach Anspruch 1, wobei das Verhältnis im Bereich von 2,5:1 bis 3,5:1 liegt.

3. Zusammensetzung nach Anspruch 2, wobei das Verhältnis im Bereich von 2,7:1 bis 3,2:1 liegt.

4. Zusammensetzung nach Anspruch 3, wobei das Verhältnis 3:1 beträgt.

5. Zusammensetzung nach irgendeinem der vorhergehenden Ansprüche, wobei das Kohlenhydrat aus Galactose, Fructose, Sucrose, Maltodextrin, raffiniertem und rohem Rohrzucker, Maissirup mit hohen Fructosegehalt, hydrolysierter Lactose, Stärken und komplexen Polysacchariden und Mischungen davon ausgewählt ist.

6. Zusammensetzung nach Anspruch 5, wobei mindestens 90 % des Galactosegehalts als Monosaccharid vorliegt.

7. Zusammensetzung nach irgendeinem der vorhergehenden Ansprüche, wobei das Koffein aus Koffein, Naturprodukten, die Koffein enthalten, und Mischungen davon ausgewählt ist.

8. Zusammensetzung nach irgendeinem der vorhergehenden Ansprüche, wobei die Menge an Phospholipid 0,1-2,0 % beträgt.

9. Zusammensetzung nach Anspruch 8, wobei die Menge an Phospholipid 0,2-1,0 % beträgt.

10. Zusammensetzung nach Anspruch 9, wobei die Menge an Phospholipid 0,2-0,3 % beträgt.

11. Zusammensetzung nach irgendeinem der vorhergehenden Ansprüche, wobei das Verhältnis von Phosphatidylcholin zu Phosphatidylserin 1:3-3:1 beträgt.

12. Zusammensetzung nach Anspruch 11, wobei das Verhältnis 1:1 beträgt.

13. Zusammensetzung nach irgendeinem der vorhergehenden Ansprüche, wobei die Fettkomponente 5-10 % einfach ungesättigter Fettsäuren und 10-15 % mehrfach ungesättigter Fettsäuren einschließt.

14. Zusammensetzung nach Anspruch 13, wobei das Verhältnis von Omega 6-Fettsäuren zu Omega 3-Fettsäuren im Bereich von 4-10:1 liegt.

15. Zusammensetzung nach irgendeinem der vorhergehenden Ansprüche, wobei das Verhältnis von Monosaccharid zur höheren Sacchariden 1:2-1:8 beträgt.

16. Zusammensetzung nach Anspruch 15, wobei das Verhältnis von Monosaccharid zur höheren Sacchariden 1:3-1:6 beträgt.

17. Zusammensetzung nach Anspruch 16, wobei das Verhältnis von Monosaccharid zur höheren Sacchariden 1:4 beträgt.

18. Zusammensetzung nach irgendeinem der vorhergehenden Ansprüche, wobei das Protein aus Molke, Kasein, einem Proteinhydrolysat und Mischungen davon ausgewählt ist.

19. Zusammensetzung nach irgendeinem der vorhergehenden Ansprüche, wobei das Protein Glutamindipeptid einschließt.

20. Zusammensetzung nach Anspruch 19, wobei die Menge an Glutamindipeptid im Bereich von 5-15 % liegt.

21. Zusammensetzung nach irgendeinem der vorhergehenden Ansprüche, die ein Antioxidationsmittel einschließt.

22. Zusammensetzung nach Anspruch 21, wobei das Antioxidationsmittel aus Ascorbinsäure, Extrakten von grünem Tee und Mischungen davon ausgewählt ist.

## Revendications

1. Composition nutritionnelle destinée à favoriser la vivacité intellectuelle, qui comprend :
un hydrate de carbone y compris le galactose,
une matière grasse,
une protéine,
de la caféine,
et d'autres ingrédients facultatifs,
dans laquelle le rapport de l'hydrate de carbone sur la protéine se trouve dans la plage de 2/1 à 4/1 en poids,
et dans laquelle la matière grasse comprend une quantité efficace de phospholipides choisis parmi; la phosphatidyl choline et la phosphatidyl sérine et des mélanges de celles-ci.

2. Composition selon la revendication 1, dans laquelle le rapport se trouve dans la plage de 2,5/1 à 3,5/1.

3. Composition selon la revendication 2, dans laquelle le rapport se trouve dans la plage de 2,7/1 à 3,2/1.

4. Composition selon la revendication 3, dans laquelle le rapport est de 3/1.

5. Composition selon l'une quelconque des revendications précédentes, dans laquelle l'hydrate de carbone est choisi parmi : le galactose, le fructose, le sucrose, la maltodextrine, le sucre de canne brut et raffiné, un sirop de glucose à haute teneur en fructose, le lactose hydrolysé, les amidons et les polysaccharides complexes et des mélanges de ceux-ci.

6. Composition selon la revendication 5, dans laquelle au moins 90 % de la teneur en galactose est sous la forme du monosaccharide.

7. Composition selon l'une quelconque des revendications précédentes, dans laquelle la caféine est choisie parmi la caféine, les produits naturels contenant de la caféine et les mélanges de ceux-ci.

8. Composition selon l'une quelconque des revendications précédentes, dans laquelle la quantité de phospholipide est de 0,1 à 2,0 %.

9. Composition selon la revendication 8, dans laquelle la quantité de phospholipide est de 0,2 à 1,0 %.

10. Composition selon la revendication 9, dans laquelle la quantité de phospholipide est de 0,2 à 0,3 %.

11. Composition selon l'une quelconque des revendications précédentes, dans laquelle le rapport de la phosphatidyl choline sur la phosphatidyl sérine est de 1/3 à 3/1.

12. Composition selon la revendication 11, dans laquelle le rapport est de 1/1.

13. Composition selon l'une quelconque des revendications précédentes, dans laquelle le composant de matière grasse comprend 5 à 10 % d'acides gras monoinsaturés et 10 à 15 % d'acides gras polyinsaturés.

14. Composition selon la revendication 13, dans laquelle la proportion d'acides gras oméga 6 sur les acides gras oméga 3 se trouve dans la plage de 4 à 10/1.

15. Composition selon l'une quelconque des revendications précédentes, dans laquelle le rapport du monosaccharide sur le saccharide supérieur est de 1/2 à 1/8.

16. Composition selon la revendication 15, dans laquelle le rapport du monosaccharide sur les saccharides supérieurs est de 1/3 à 1/6.

17. Composition selon la revendication 16, dans laquelle le rapport du monosaccharide sur les saccharides supérieurs est de 1/4.

18. Composition selon l'une quelconque des revendications précédentes, dans laquelle la protéine est choisie parmi : le lactosérum, la caséine, un hydrolysat de protéine et les mélanges de ceux-ci.

19. Composition selon l'une quelconque des revendications précédentes, dans laquelle la protéine comprend le dipeptide glutamine.

20. Composition selon la revendication 19, dans laquelle la quantité de dipeptide glutamine se trouve dans la plage de 5 à 15 %.

21. Composition selon l'une quelconque des revendications précédentes, comprenant un antioxydant.

22. Composition selon la revendication 21, dans laquelle l'antioxydant est choisi parmi l'acide ascorbique, les extraits de thé vert et les mélanges de ceux-ci.
